Europäisches Patentamt

European Patent Office

Office européen des brevets

(11) Publication number: **0 252 333**
**A1**

# EUROPEAN PATENT APPLICATION

(21) Application number: 87108525.4

(22) Date of filing: 12.06.87

(51) Int. Cl.⁴: **C12M 3/00** , C12M 1/34

(30) Priority: 12.06.86 PL 260076

(43) Date of publication of application:
**13.01.88 Bulletin 88/02**

(84) Designated Contracting States:
**AT BE CH DE ES FR GB GR IT LI LU NL SE**

(71) Applicant: **Pomorska Akademia Medyczna**
**ul. Rybacka 1**
**PL-70952 Szczecin(PL)**

(72) Inventor: **Tyrakowski, Tomasz**
**61-245 Poznan**
**Os.Rusa 50 m 6(PL)**
Inventor: **Mikulski, Marek**
**70-123 Szczecin**
**ul. Dunikowskiego 6(PL)**

(74) Representative: **Schrimpf, Robert et al**
**Cabinet Regimbeau 26, Avenue Kléber**
**F-75116 Paris(FR)**

(54) **A method of the biological culture of cells and tissues and a means of implementing that method.**

(57) The invention solves the problem of an effective biological culture of tissues and of coating filterable bases with those tissues. According to the method as per this present invention the cultures in the form of tissue sections 1 are set at the bottom of filterable membrane 2 in the medium of a stationary nutrient fluid, the other side of the bottom of that filterable membrane being washed with a slow stream of the nutrient fluid.

A means for the biological culture of cells and tissues comprises filterable membrane 2 on which are set tissue sections 1 and is characterised in that the filterable membrane devides into parts tight chamber 3 filled with the nutrient fluid, nutrient fluid delivery 7 and drain 8 pipes leading to and from each part of chamber 3.

Fig.1

# A method of the biological culture of cells and tisures and a means of implementing that method

The subject of this present invention is a method of the biological culture of cells and tissues and a means of implementing that method.

The aim of the cultrue of cells, tissues or limbs is to provide conditions and to start and carry on the biological processes that enable that biological material to retain its internal structure and, at least to a definite degree, to retain its function, whereas the most advantageous aim is to differentiate and to grow the components of that material. A method is known from US Patent Specification No. 4,200,689 for the biological culture of cells and a means of implementing that method. This known means has a middle transparent tubular member to ether end of which has been attached an extreme Y-shaped member. The extreme members are connected to the ends of the middle tubular member with sleeves, and that connection is detachable in nature. Inside the means, two bundles of capillary fibres are located to form two perfusion circuits, the bundles being arranged together inside the middle member and the inlets and outlets of the bundles being in the arms of the extreme members.

The shell of the middle tubular member has holes with secured attaching pipes through which cells are introduced to provide the beginning of a later culture of cells fed by means of perfusion circuits.

From the publication In Vitro, vol. 20, No. 3, Part I, March 1984, a means is also known for biological culture, whose basic component is a foamed cellulose disc having two symmetrically spaced holes having their axes parallel to the axis of the disc. Each of these holes is covered from one side with a filterable collagen membrane and the whole disc is placed in a recess of a moulded plastics base and covered with an also moulded plastics cover. Each of the recesses of the base containing the discs is provided with holes that discrease the filterable membrane screening the holes of the disc. Completed sets of the discs are immersed in a Leighton flask/vessel/filled with a liquid.

The procedure involved in biological culture with the application of that known means is to wash the foamed discs with water and with acetone and an alcohol and, then, to impregnate them with a collagen nutrient diluted with pure methyl alcohol.

In the discs thus prepared biological cultrues are set at the bottom of the filterable collagen diaphragms and on nutrient base, the discs being put into the plastics base which is then inverted and closed from its top with a cover and, finally the whole is immersed in a Leighton flask/vessel/ filled with a liquid.

The essence of this present invention involves feeding the cultures set on the filterable membrane through the latter with a nutrient fluid in such a manner that the cultures are set at the bottom of the filterable membrane in the medium of an stationary nutrient fluid, whereas the other side of the bottom of that diaphragm is washed with a slow stream of the nutrient fluid.

A successive essential feature of the method as per this present invention involves developing hydrostatic pressure in the fluid filling that part of the chamber.

The effect of employing the method and means as per this present invention is a marked increase in the degree of growth of the tissues of the culture. This increase in the tissue growth results from the tissues being grown are better and more intensively fed, and the toxic products of metabolic transformations are rapidly and effectively removed from the region where those metabolic transformations take place.

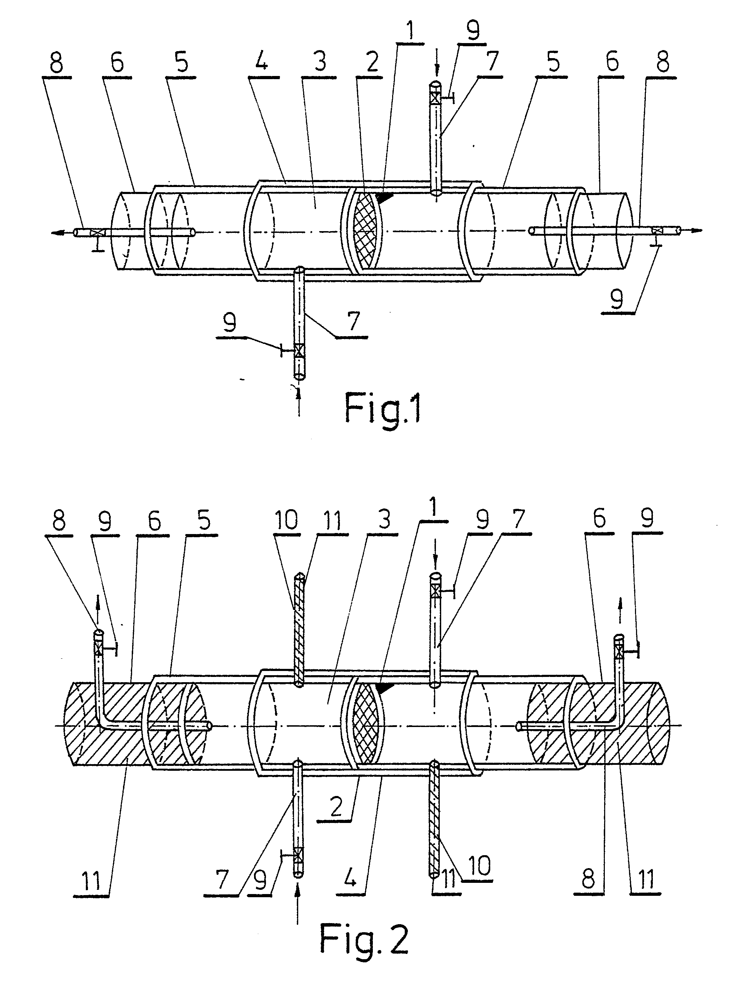

The subject of this present invention is shown in embodiments, the means as per this present invention being illustrated in the drawing whose Fig. 1 shows a perspective view of the first example of the means made of a transparent plastic material and Fig. 2 also shows a perspective view of the second somewhat different example of that means.

The biological culture is grown in such a manner that tissue section 1 taken by, for example, biopsy is prepared as the culture and, next, that section is rested on filterable diaphragm 2. The area adherring to filterable diaphragm 2 and comprising tissue section 1 set on that diaphragm is filled with a nutrient fluid which subsides and is left in that subsided condition.

Filterable diaphragm 2 is, on the other hand, washed with a jet of the nutrient fliud and the motion of that jet is adjusted depending on the conditions of growing that tissue culture.

For determining the effect of hydrostatic pressure on the cell culture being grown use is made of an additional phase, where that culture is grown under increased hydrostatic pressure of stepwise values.

Hydrostatic pressure is developed by known methods and means and it is transmitted and applied via supply pipes 7 with valves 9 almost closed on drain pipes 8.

An example of the possible embodiment of the means as per this present invention is shown in Fig. 1 illustrating cylindrical chamber 3 made of transparent polyethylene components and set up from outer cylinder 4 and successive inner cylinders 5 that are pressed into that cylinder from either side, have a somewhat smaller diameter and are closed with 6 on either side.

At the middle of chamber 3 there filterable membrane 2 secured square with the chamber axes and at the upper region and on that membrane there is set tissue section 1. In both the parts of chamber and in its lateral surfaces there are holes in which are secured delivery pipes 7, drain pipes 8 being secured in the holes made in plugs 6 and these pipes are arranged along the axis of chamber 3.

Delivery 7 and drain 8 pipes are provided with valves 9 adjusting or cutting off the flow of the nutrient fluid.

The setting up of the chamber 3 of the means from the components described in this embodiment simplifies the assembly of the means since that assembly involves pressing the first of inner cylinders 5 down half the length of outer cylinder 4, fitting filterable membrane 2 and, finally pressing in another inner closing cylinder 5.

According to the second embodiment of the means as per this present invention that means is set up from component parts, such as those of the first embodiment, and from additional components. These additional components which the means is composed of are measuring pipes 10 mounted in the holes made in the lateral sides of both the parts of chamber 3 and preferably aligned with delivery pipes 7.

Measuring pipe 10 is filled with gel 11, plug 6 being also filled with it and at least part of the surface of that gel in measuring pipes 10 and plugs 6 remaining directly in contact with the surface of the nutrient fluid filling both the parts of chamber 3.

Gel 11 filling measuring pipes 10 and plugs 6 is characterised by good electrical conductance and is connected to the ends of the measuring circuit during electrical measurements. These measurments involve mainly determining the resistance of the medium where cells are grown, disclosing the presence of the electrical potential developed by that culture and measuring its value.

The invention finds application in the biological culture of tissues and, particularly, in the culture of tissues on filterable bases, and in short of coating these bases with the biological structure of a tissue.

able membrane 2 in the medium of a stationary nutrient fluid, the other side of that bottom of the filterable membrane being washed with a slow stream of the nutrient fluid.

2. A method of the biological culture as per Claim 1, characterised in that hydrostatic pressure is developed in the nutrient fluids adjoining either side of filterable membrane 2.

3. A method of the biological culture as per Claim 1, characterised in that the culture is prepared by biopsy in the form of tissue section 1.

4. A means intended for the biological culture of cells and tissues and comprising a filterable membrane on which are set the cultures in the form of tissue sections, characterised in that filterable membrane 2 on one side of which is set the culture in the form of tissue sections 1 divides into parts chamber 3 filled with a nutrient filter, nutrient fluid delivery 7 and drain 8 pipes leading to and from each part of chamber 3.

5. A means for the biological culture as per Claim 4, characterised in that the individual parts of chamber 3 partitioned with filterable mem brane 2 comprises spaces filled with gel 11 of definite electrical conductance, the surface of each of the spaces being at least partially filled with that gel remaining in contact with the nutrient fluid filling the given part of chamber 3.

6. A means for the biological culture as per Claim 4 characterised in that chamber 3 is set up from outer cylinder 4 and inner cylinders 5 which have been pressed into the former on either side, are of smaller diameter and are closed with plugs 6, the lateral surfaces of chamber 3 having delivery pipes 7 secured thereto and connected to its inside, plugs 6 having drain pipes 8 mounted therein and also connected to the inside of the chamber, and filterable membrane 2 being secured at the middle of the chamber and square with its axis.

## Claims

1. A method of the bioligical culture of cells and tissues during which the cells and tissues are fed with a nutrient fluid, characterised in that the cultures of tissue 1 are set at the bottom of filter-

0 252 333

Fig.1

Fig.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A- 839 245 (DR.MED.E. KANZ) * Figures; claims 1-16; page 2, lines 28-32; page 2, line 108 - page 3, line 69 * | 1-4 | C 12 M 3/00<br>C 12 M 1/34 |
| Y | | 5 | |
| A | | 6 | |
| Y | FR-A-2 228 839 (BACTOMATIC INC.) * Claims 1,5,7; page 13, lines 9-22; figures 11,12 * | 5 | |
| P,X | US-A-4 636 473 (C. KLEINSTREUER) * Figures; column 2, line 66 - column 3, line 68 * | 1-4 | TECHNICAL FIELDS SEARCHED (Int. Cl.4) |
| A | | 6 | C 12 M |

The present search report has been drawn up for all claims

| Place of search | Date of completion of the search | Examiner |
|---|---|---|
| THE HAGUE | 12-10-1987 | COUCKE A.O.M. |